(19) Europäisches Patentamt
European Patent Office
Office européen des brevets

(11) Veröffentlichungsnummer: **0 065 712**
**B1**

(12) # EUROPÄISCHE PATENTSCHRIFT

(45) Veröffentlichungstag der Patentschrift: **22.07.87**

(21) Anmeldenummer: **82104171.2**

(22) Anmeldetag: **13.05.82**

(51) Int. Cl.⁴: **C 07 C 103/365,** C 07 C 103/37, C 07 C 103/375, C 07 C 103/38, C 07 C 103/42, C 07 D 211/70, C 07 D 307/52, C 07 D 309/32, A 01 N 37/22, A 01 N 43/08, A 01 N 43/16

(54) **N-(1-Alkenyl)-chloressigsäureanilide, Verfahren zu ihrer Herstellung sowie ihre Verwendung als Herbizide und Pflanzenwachstumsregulatoren.**

(30) Priorität: **26.05.81 DE 3120990**

(43) Veröffentlichungstag der Anmeldung: **01.12.82 Patentblatt 82/48**

(45) Bekanntmachung des Hinweises auf die Patenterteilung: **22.07.87 Patentblatt 87/30**

(84) Benannte Vertragsstaaten: **BE CH DE FR GB IT LI**

(56) Entgegenhaltungen:
**EP-A-0 010 972**
**EP-A-0 018 509**
**EP-A-0 037 019**
**DE-A-2 042 497**
**DE-A-2 835 157**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Thomas, Rudolf, Dr., Dellbusch 269, D-5600 Wuppertal 2 (DE)**
Erfinder: **Eue, Ludwig, Dr., Paul- Klee- Strasse 36, D-5090 Leverkusen 1 (DE)**
Erfinder: **Schmidt, Robert, Dr., Im Waldwinkel 110, D-5060 Bergisch Gladbach 2 (DE)**
Erfinder: **Lürssen, Klaus, Dr., August- Kierspel- Strasse 89, D-5060 Bergisch Gladbach 2 (DE)**

**EP 0 065 712 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue N-(1-Alkenyl)-chloressigsäureanilide, ein Verfahren zu ihrer Herstellung sowie ihre Verwendung als Herbizide und als Pflanzenwachstumsregulatoren.

Es ist bereits bekannt geworden, daß zahlreiche Chloracetanilide herbizide Eigenschaften besitzen (vgl.EP-A-0 037 019, DE-A-2 835 157 und EP-A-0 010 972). So läßt sich beispielsweise 2,6-Diethyl-N-methoxymethyl-chloracetanilid zur Unkrautbekämpfung verwenden (vgl. US-PS 3 442 945). Die Wirkung dieser Verbindungen ist jedoch nicht immer voll befriedigend, insbesondere ist der Einsatz als selektive Herbizide bei vielen Kultur-Pflanzen nur begrenzt möglich. Weiterhin bekannt sind N(1,3- Alkadienyl)-Carbonylverbindungen (vgl. DE-OS 2 042 497).

Es wurden nun neue N-(1-Alkenyl)-chloressigsäureanilide der Formel

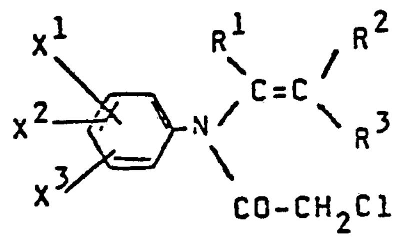

(I)

in welcher

$R^1$ für Wasserstoff oder geradkettiges oder verzweigtes Alkyl mit 1 bis 4 Kohlenstoffatomen steht,

$R_2$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl steht,

$R^3$ für Wasserstoff, geradkettiges oder verzweigtes Alkyl mit 1 bis 4 Kohlenstoffatomen, Alkoxy mit 1 bis 4 Kohlenstoffatomen, Alkoxyalkyl mit 1 bis 4 Kohlenstoffatomen im Alkylteil und 1 bis 4 Kohlenstoffatomen im Alkoxyteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkylteil steht, oder

$R^1$ und $R^2$ gemeinsam mit der C=C-Doppelbindung für einen gegebenenfalls ein- oder mehrfach substituierten, einfach oder mehrfach ungesättigten, 5- oder 6-gliedrigen Ring stehen, der ein oder zwei Heteroatome und/oder eine Carbonyl-Gruppe enthalten kann, wobei als Substituenten beispielsweise genannt seien: Alkyl mit 1 bis 4 Kohlenstoffatomen, Halogen und Hydroxy;

oder

$R^2$ und $R^3$ gemeinsam mit dem angrenzenden Kohlenstoffatom für einen gegebenenfalls ein- oder mehrfach substituierten, gesättigten oder ungesättigten, 5- oder 6-gliedrigen Ring stehen, der ein oder zwei Heteroatome enthalten kann, wobei als Substituenten beispielsweise genannt seien: Alkyl mit 1 bis 4 Kohlenstoffatomen, Halogen und Hydroxy;

$X^1$ für Wasserstoff, geradkettiges oder verzweigtes Alkyl mit 1 bis 4 Kohlenstoffatomen, Fluor, Chlor oder Brom steht,

$X^2$ für Wasserstoff, geradkettiges oder verzweigtes Alkyl mit 1 bis 4 Kohlenstoffatomen, Fluor, Chlor oder Brom steht, und

$X^3$ für Wasserstoff und geradkettiges Alkyl mit 1 bis 4 Kohlenstoffatomen, Fluor, Chlor oder Brom steht,

gefunden.

Weiterhin wurde gefunden, daß man die N-(1-Alkenyl)-chloressigsäureanilide der Formel (I) erhält, wenn man Azine der Formel

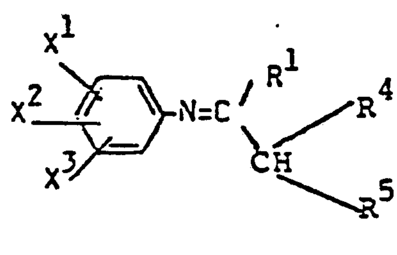

(II)

in welcher

$R^1$, $X^1$, $X^2$ und $X^3$ die oben angegebene Bedeutung haben,

$R^4$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl steht;

$R^5$ für Wasserstoff, geradkettiges oder verzweigtes Alkyl mit 1 bis 4 Kohlenstoffatomen, Alkoxy mit 1 bis 4

Kohlenstoffatomen, Alkoxyalkyl mit 1 bis 4 Kohlenstoffatomen im Alkylteil und 1 bis 4 Kohlenstoffatomen im Alkoxyteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkoxyteil steht oder

$R^1$ und $R^4$ gemeinsam mit der angrenzenden C-C-Bindung für einen gegebenenfalls ein- oder mehrfach substituierten, einfach oder mehrfach ungesättigten, 5- oder 6-gliedrigen Ring stehen; der ein oder zwei Heteroatome und/oder eine Carbonyl-Gruppe enthalten kann, wobei als Substituenten beispielsweise genannt seien: Alkyl mit 1 bis 4 Kohlenstoffatomen, Halogen und Hydroxy;

oder

$R^4$ und $R^5$ gemeinsam mit dem angrenzenden Kohlenstoffatom für einen gegebenenfalls ein- oder mehrfach substituierten, gesättigten oder ungesättigten 5- oder 6-gliedrigen Ring stehen, der ein oder zwei Heteroatome enthalten kann, wobei als Substituenten beispielsweise genannt seien: Alkyl mit 1 bis 4 Kohlenstoffatomen, Halogen und Hydroxy;,

mit Chloracethalogeniden der Formel

$Hal\text{-}CO\text{-}CH_2\text{-}Cl$ (III)

in welcher

Hal für Chlor oder Brom steht,

gegebenenfalls in Gegenwart eines Verdünnungsmittels umsetzt und die entstehenden Chloracetanilide der Formel

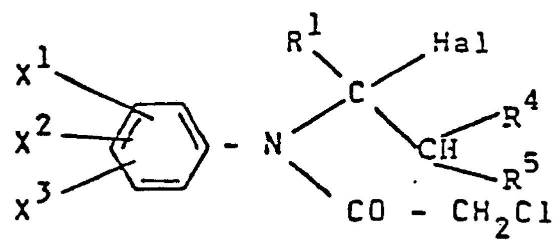

(IV)

in welcher

$R^1$, $R^4$, $R^5$, $X^1$, $X^2$, $X^3$ und Hal die oben angegebene Bedeutung haben,

ohne vorherige Isolierung, gegebenenfalls in Gegenwart einer Base, der Halogenwasserstoffeliminierung unterwirft.

Außerdem wurde gefunden, daß die N-(1-Alkenyl)-chlor-essigsäureanilide der Formel (I) gute herbizide, insbesondere selektiv-herbizide Eigenschaften aufweisen. Darüber hinaus lassen sie sich sehr gut zur Regulierung des Pflanzenwachstums verwenden.

Überraschenderweise zeigen die erfindungsgemäßen Verbindungen gegenüber dem bekannten 2,6-Diethyl-N-methoxymethylchloracetanilid, welches chemisch und wirkungsmäßig eine naheliegende Verbindung ist, bei vergleichbar guter allgemeiner Unkrautwirkung bessere Möglichkeiten zum Einsatz als selektive Unkrautbekämpfungsmittel. Ferner besitzen die erfindungsgemäßen Stoffe überraschenderweise auch eine starke pflanzenwuchsregulierende Wirksamkeit.

Die erfindungsgemäßen N-(1-Alkenyl)-chloressigsäureanilide sind durch die Formel (I) allgemein definiert. In dieser Formel stehen vorzugsweise

$R^1$ für Wasserstoff, Methyl, Ethyl, Isopropyl oder tert.-Butyl,

$R^2$ für Wasserstoff, Methyl, Ethyl, Isopropyl, Methoxy, Ethoxy, Isopropoxy, Methoxymethyl, Ethoxymethyl, iso-Hydroxypropyl oder Benzyl;

$R^3$ für Wasserstoff, Methyl, Ethyl, Isopropyl, Methoxy, Ethoxy, Isopropoxy, Methoxymethyl, Ethoxymethyl, Methylcarbonyl, Ethylcarbonyl, Methylcarbonylmethyl, Methoxycarbonyl, Ethoxycarbonyl, Methoxycarbonylmethyl,

oder

$R^1$ und $R^2$ gemeinsam mit der C=C-Doppelbindung für einen gegebenenfalls durch Methyl, Ethyl, Hydroxy und/oder Chlor substituierten, einfach oder mehrfach ungesättigten 5- oder 6-gliedrigen Ring, der ein oder zwei Heteroatome, wie zum Beispiel Sauerstoff, Stickstoff und/oder Schwefel und/oder eine Carbonyl-Gruppe, enthalten kann;

oder

$R^2$ und $R^3$ gemeinsam mit dem angrenzenden Kohlenstoffatom für einen gegebenenfalls durch Methyl, Ethyl, Hydroxy und/oder Chlor substituierten, gesättigten oder ungesättigten 5- oder 6-gliedrigen Ring, der ein oder zwei Heteroatome, wie zum Beispiel Sauerstoff, Stickstoff und/oder Schwefel, enthalten kann;

$X^1$ für Wasserstoff, Methyl, Ethyl, Isopropyl, Isobutyl, sek.-Butyl, tert.-Butyl, Chlor oder Fluor;

$X^2$ für Wasserstoff, Methyl, Ethyl, Isopropyl, Isobutyl, sek.-Butyl, tert.-Butyl, Chlor oder Fluor;

und

$X^3$ für Wasserstoff, Methyl, Ethyl, Isopropyl, Isobutyl, sek.-Butyl, tert.-Butyl, Chlor oder Fluor.

Verwendet man beispielsweise N-Ethyliden-2,6-diethylanilin und Chloracetylchlorid als Ausgangsstoffe, so

kann der Reaktionsablauf nach dem erfindungsgemäßen Verfahren durch das folgende Formelschema wiedergegeben werden:

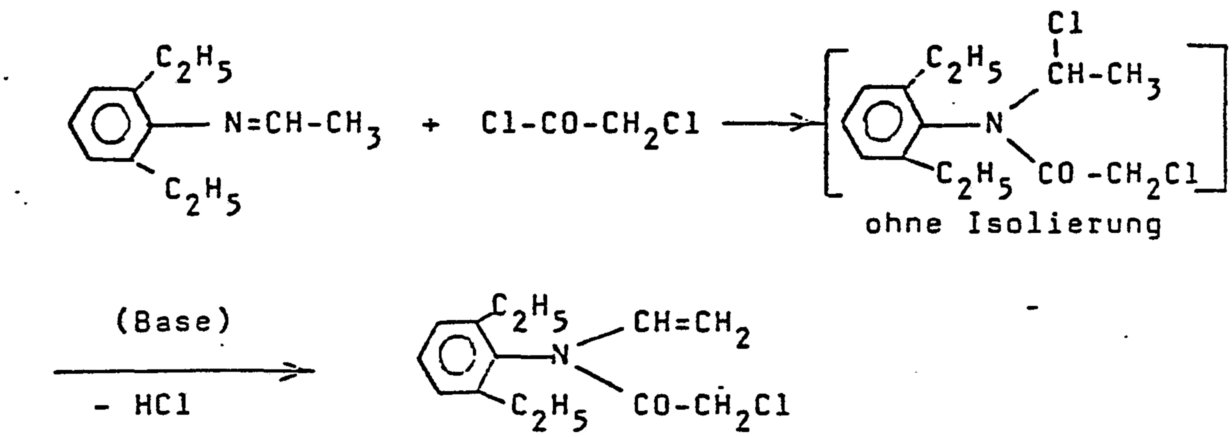


Verwendet man beispielsweise N-(3-Cyclohexenyl-methyliden)-2,6-dimethylanilin und Chloracetylchlorid als Ausgangsstoffe, so kann der Reaktionsablauf nach dem erfindungsgemäßen Verfahren durch das folgende Formelschema wiedergegeben werden:

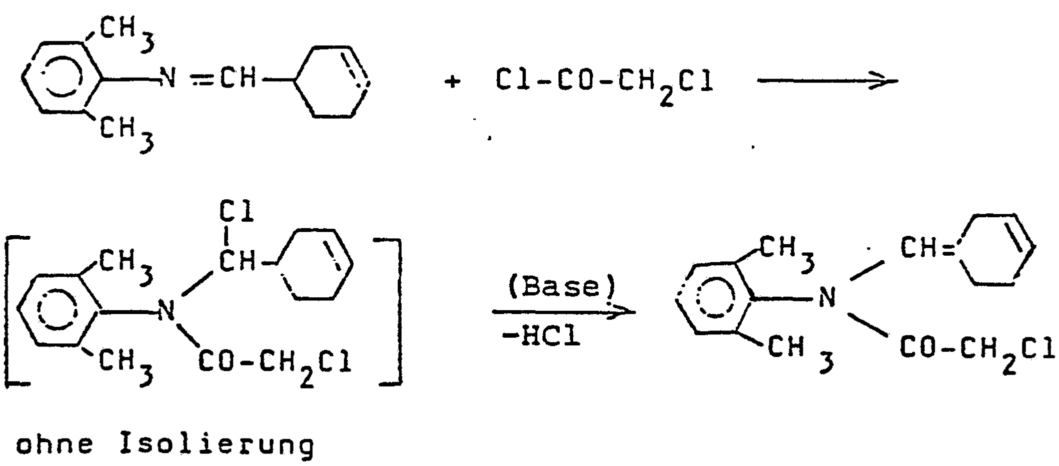


Verwendet man beispielsweise N-(Cyclopentyliden)-2-ethyl-6-methylanilin und Chloracetylchlorid als Ausgangsstoffe, so kann der Reaktionsablauf nach dem erfindungsgemäßen Verfahren durch das folgende Formelschema wiedergegeben werden:

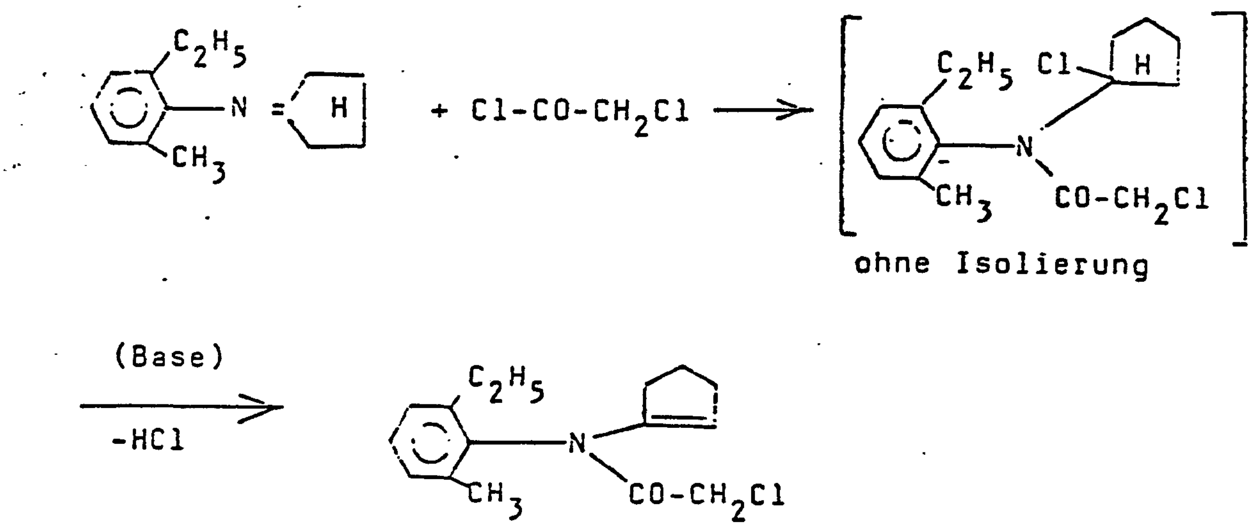


Die für die erfindungsgemäße Reaktion als Ausgangsstoffe zu verwendenden Azine sind durch die Formel (II) allgemein definiert.

Bevorzugte Ausgangsstoffe sind solche Verbindungen der Formel (II), in denen $R^1$, $X^1$, $X^2$ und $X^3$ diejenigen Bedeutungen haben, die bereits im Zusammenhang mit der Beschreibung der erfindungsgemäßen Stoffe der Formel (I) für diese Reste als bevorzugt genannt wurden, und ferner

$R^4$ für Wasserstoff, Methyl, Ethyl, Isopropyl, Methoxy, Ethoxy, Isopropoxy, Methoxymethyl, Ethoxymethyl, iso-Hydroxypropyl oder Benzyl steht;

$R^5$ für Wasserstoff, Methyl, Ethyl, Isopropyl, Methoxy, Ethoxy, Isopropoxy, Methoxymethyl, Ethoxymethyl, Methylcarbonyl, Ethylcarbonyl, Methylcarbonylmethyl, Methoxycarbonyl, Ethoxycarbonyl, Methoxycarbonylmethyl steht;

oder

$R^1$ und $R^4$ gemeinsam mit der angrenzenden C-C-Bindung für einen gegebenenfalls durch Methyl, Ethyl, Hydroxy und/oder Chlor substituierten, gesättigten oder ungesättigten 5- oder 6-gliedrigen Ring stehen, der ein oder zwei Heteroatome, wie zum Beispiel Sauerstoff, Stickstoff und/oder Schwefel und/oder eine Carbonyl-Gruppe, enthalten kann;

oder

$R^4$ und $R^5$ gemeinsam mit dem angrenzenden Kohlenstoffatom für einen gegebenenfalls durch Methyl, Ethyl, Hydroxy und/oder Chlor substituierten, gesättigten oder ungesättigten 5- oder 6-gliedrigen Ring stehen, der ein oder zwei Heteroatome, wie zum Beispiel Sauerstoff, Stickstoff und/oder Schwefel, enthalten kann;

Die Azine der Formel (II) sind bekannt oder lassen sich in einfacher Weise nach im Prinzip bekannten Verfahren herstellen (vgl. DE-OS 16 70 859 und DE-OS 30 11 084). So lassen sich die Azine der Formel (II) dadurch synthetisieren, daß man Aniline der Formel

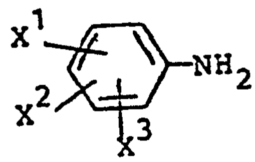
(V)

in welcher

$X^1$, $X^2$ und $X^3$ die oben angegebene Bedeutung haben,

mit Aldehyden oder Ketonen der Formel

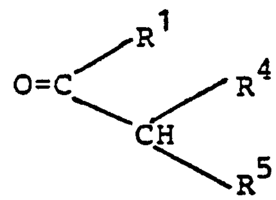

(VI)

in welcher

$R^1$, $R^4$ und $R^5$ die oben angegebene Bedeutung haben,

wobei die Verbindungen der Formel (VI) gegebenenfalls auch in Form ihrer Acetale bzw. Ketale vorliegen können,

gegebenenfalls in Gegenwart eines inerten organischen Lösungsmittel, wie beispielsweise Methylenchlorid oder Toluol, und gegebenenfalls in Gegenwart eines basischen oder sauren Katalysators, wie beispielsweise Dimethylbenzylamin oder p-Toluolsulfonsäure, bei einer Temperatur zwischen -10°C und +120°C umsetzt (vergleiche auch die Herstellungsbeispiele).

Die weiterhin bei dem erfindungsgemäßen Verfahren als Ausgangsstoffe benötigten Chloracethalogenide sind durch die Formel (III) definiert. Die betreffenden Chloracethalogenide sind bekannt.

Als Verdünnungsmittel kommen für das erfindungsgemäße Verfahren inerte organische Lösungsmittel in Frage. Hierzu gehören vorzugsweise Ketone, wie Aceton und Methylethylketon; Nitrile, wie Acetonitril; Ether, wie Tetrahydrofuran oder Dioxan; Ester, wie Essigester; halogenierte Kohlenwasserstoffe, wie Methylenchlorid; sowie insbesondere aromatische Kohlenwasserstoffe, wie Benzol und Toluol.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen -20 und +150°C, vorzugsweise zwischen -20 und +100°C.

Die Halogenwasserstoffeliminierung in der zweiten Stufe des erfindungsgemäßen Verfahrens erfolgt in allgemein üblicher und bekannter Weise. Man verfährt in der Regel in der Weise, daß man das Reaktionsgemisch ohne vorherige Isolierung der Stoffe der Formel (IV) erhitzt oder bei den oben genannten Temperaturen eine Base zugibt.

Als Basen können hierbei alle üblicherweise für derartige Umsetzungen geeigneten Säureakzeptoren eingesetzt werden. Vorzugsweise verwendbar sind Alkalicarbonate, wie Natrium- und Kaliumcarbonat oder Natriumhydrogencarbonat; ferner niedere tertiäre Alkylamine, Aralkylamine, aromatische Amine oder Cycloalkylamine, wie z. B. Triethylamin, Dimethylbenzylamin, Pyridin, 1,5-Diazabicyclo-[4.3.0]-non-5-en (DBN) und 1,8-Diaza-bicyclo-[5.4.0]-undec-7-en (DBU). Es ist aber auch möglich, einen entsprechenden Überschuß an Azin der Formel (II) einzusetzen.

Die Reaktionstemperaturen können bei der Durchführung der Halogenwasserstoffeliminierung in gleicher Weise variiert werden, wie es für die erste Stufe des erfindungsgemäßen Verfahrens beschrieben wurde.

Bei der Durchführung des erfindungsgemäßen Verfahrens setzt man die Ausgangsstoffe der Formeln (II) und (III) vorzugsweise in äquimolaren Mengen ein. Die Isolierung der Endprodukte erfolgt nach üblichen Methoden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellarium, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischeamum, Sphenoclea, Dactyloctenium, Agrotis, Alopecurus, Apera.

Monokotyle Kulturen der Gattungen: Oryza, Zea, Triticum, Hordeum, Avena, Secala, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Wein-, Citrus-, Nuss-, Bananen-, Kaffee-, Tee-, Gummi-, Oelpalm-, Kakao-, Beerenfrucht- und Hopfenanlagen und zur selektiven

Unkrautbekämpfung in einjährigen Kulturen eingesetzt werden.

Die erfindungsgemäßen Wirkstoffe zeigen neben einer sehr guten allgemeinen herbiziden Wirkung eine gute Verträglichkeit gegenüber Nutzpflanzen. So ist es möglich, wichtige Schadgräser selektiv in wichtigen Nutzpflanzenkulturen, wie z. B. Baumwolle, Mais, Sojabohnen und Rüben, zu bekämfen.

Die erfindungsgemäßen Wirkstoffe greifen in den Metabolismus der Pflanzen ein und können deshalb als Wachstumsragulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmnerstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pfanzenteilen.

Mit Wachstumsregulatoren läßt sich häufig auch eine Förderung des vegetativen Wachstums erzielen. Dies ist von großem Nutzen, wenn die vegetativen Pflanzerteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Anderseits ist aber auch möglich, das Wachstum der Seitentriebe zu hemmen. Für diese Wirkung besteht z. B. größes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einen gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Bluten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des

Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, vie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziet werden. Hierdurch wird die Kultivierung von Pflanzen in Gebieten möglich, die hierzu normalerweise ungeeignet sind.

Die Wirkstoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch vermischen der Wirkstoffe mit Streckmitteln, also flüssigen Lösungsmitteln, unter Druch stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z. B. gebrochene und fraktionierte natürliche Gesteine vie Calcit, Marmor, Bims, Sepiolith, Dolomit sowie synthetische Granulate aus anorganischen und organischen Mehlen sowie Granulate aus organischem Material vie Sägemehl, Kokosnußschalen, Maiskolben und Tabakstengel. Als Emulgier- und / oder schaumerzeugende Mittel kommen in Frage: z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet verden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen, oder Stäuben.

Bei der Anwendung der erfindungsgemäßen Wirkstoffe als Herbizide können die Wirkstoffe sowohl vor als auch nach dem Auflaufen der Pflanzen appliziert werden.

Die Anwendung wird vorzugsweise vor dem Auflaufen der 2Pflanzen, also im pre-emergence-Verfahren, vorgenommen. Sie können auch vor der Saat in den Boden eingearbeitet werden.

Die Aufgewandte Wirkstoffmenge kann in größeren Bereichen schwanken. Sie hängt im wesentlichen von der Art des gewünschten Effekts ab. Im allgemeinen liegen die Aufwandmengen beim Einsatz der erfindungsgemäßen Verbindungen als Herbizide zwischen 0,05 und 10 kg Wirkstoff pro ha, vorzugsweise zwischen 0,1 und 5 kg/ha; und beim Einsatz als Pflanzenwachstumsregulatoren zwischen 0,01 und 50 kg

Wirkstoff pro ha; vorzugsweise zwischen 0,05 und 10 kg/ha.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

In entsprechenden Aufwandmengen zeigen die erfindungsgemäßen Verbindungen auch fungizide Wirkung, wie insbesondere gegen Oomyceten bzw. gegen Getreidekrankheiten. Dabei entfalten sie nicht nur eine protektive, sondern teilweise auch systemische Wirkung. So gelingt es, Pflanzen gegen Pilzbefall zu schützen, wenn man die Wirkstoffe uber den Boden und die Wurzel oder über das Saatgut den oberirdischen Teilen der Pflanze zuführt.

Herstellung und Verwendung der erfindungsgemäßen Wirkstoffe gehen aus den nachfolgenden Beispielen hervor.

## Herstellungsbeispiele

**Beispiel 1**

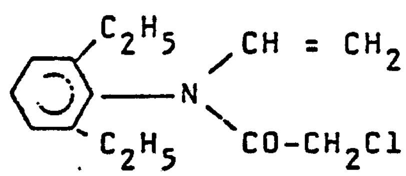

Zu einer Lösung von 113 g (1 Mol) Chloracetylchlorid in 150 ml Toluol werden bei -10 bis 0° C 175 g (1 Mol) N-Ethyliden-2,6-diethylanilin getropft. Man läßt 3 Stunden bei 0° C nachrühren und tropft dann eine Lösung von 101g Triethylamin in 500 ml Toluol zu, wobei die Temperatur durch Kühlen bei -10 bis 0° C gehalten wird. Man läßt 2 Stunden bei Raumtemperatur nachrühren, saugt das ausgefallene Triethylamin-hydrochlorid ab, wäscht mit 100 ml Toluol nach und engt die organische Phase im Vakuum ein. Der dabei erhaltene ölige Rückstand wird durch Destillation oder fraktionierte Kristallisation aus Petrolether weiter gereinigt.

Man erhält 190g (76 % der Theorie) N-Vinyl-N-(2,6-diethyl-phenyl)-chloracetamid als hellgelbes Oel vom Siedepunkt 97-100° C/0,1 mbar, das langsam durchkristallisiert (Schmelzpunkt 45-47° C).

**Beispiel 2**

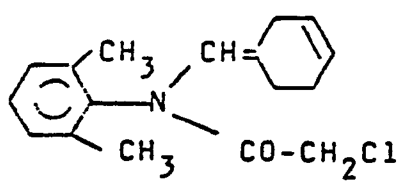

Zu einer Lösung von 22,6g (0,2 Mol) Chloracetylchlorid in 50 ml Toluol werden bei 20° C unter Rühren 42,6g (0,2 Mol) N-(3-Cyclohexenyl-methyliden)-2,6-dimethylanilin getropft. Man rührt 15 Stunden nach und tropft dann bei 20° C 20,2g (0,2 Mol) Triethylamin zu. Es wird 15 Stunden bei 20° C nachgerührt; dann wird der Niederschlag abfiltriert. Man wäscht die organische Phase zunächst mit verdünnter, wäßriger Bicarbonatlösung, dann mit Wasser und engt schließlich im Vakuum ein. Der Rückstand wird mehrfach mit Petrolether extrahiert. Die vereinigten Extrakte werden im Vakuum eingeengt.

Man erhält 30,3g (52,3 % der Theorie) N-(3-Cyclohexenylidenmethyl)-N-(2,6-dimethylphenyl)-chloracetamid in Form eines schwach gelben Oels.

**Beispiel 3**

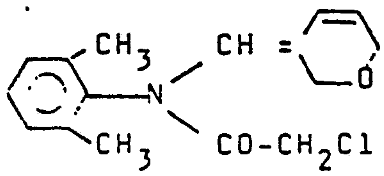

Zu einer Lösung von 22,4g (0,2 Mol) Chloracetylchlorid in 30 ml Toluol werden bei 20°C unter Rühren 43g (0,2 Mol) N-(Z,3-Dihydro-α-pyran-5-yl-methyliden)-2,6-dimethyl-anilin getropft. Man läßt 2 Stunden bei 20°C nachrühren und tropft dann bei 20°C 20,2g (0,1 Mol) Triethylamin zu. Nach 1 Stunde Nachrührzeit bei 20°C saugt man vom ausgefallenen Triethylamin-hydrochlorid ab, wäscht das Filtrat zunächst mit verdünnter, wäßriger Bicarbonatlösung, dann mit Wasser und engt schließlich im Vakuum ein. Der Rückstand wird aus Petrolether umkristallisiert.

Man erhält 38,2g (65,4 % der Theorie) N-(2,3-Dihydro-α-pyran-5-ylidenmethyl)-N-(2,6-dimethylphenyl)-chloracetamid in Form farbloser Kristalle von Schmelzpunkt 80°C.

**Beispiel 4**

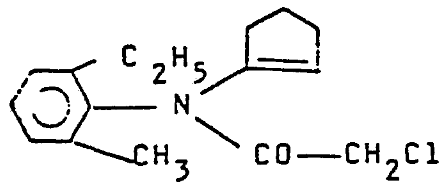

Zu einer Lösung von 11,3g (0,1 Mol) Chloracetylchlorid in 10 ml Toluol werden unter Rühren bei 20°C 20,1 g (0,1 Mol) N-(Cyclopentyliden)-2-ethyl-6-methyl-anilin getropft. Man läßt 2 Stunden bei 20°C nachrühren und tropft dann 15,2 g (0,1 Mol) Diaza-bicyclo-undecan zu. Man läßt 1 Stunde bei 20°C nachrühren und gießt das Reaktionsgemisch in 200 ml verdünnte, wäßrige Bicarbonatlösung. Die organische Phase wird abgetrennt, mehrfach mit Wasser gewaschen und dann im Vakuum eingedampft. Zur Entfernung von Anilinresten wird dann im Hochvakuum andestilliert.

Man erhält 15,7g (56,7 % der Theorie) N-(1-Cyclopentenyl)-N-(2-ethyl-6-methyl-phenyl)-chloracetamid in Form eines gelben Öles.

**Beispiel 5**

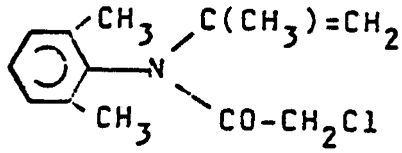

Zu einer Lösung von 113g (1 Mol) Chloracetylchlorid in 100 ml Toluol werden 322,5g (2 Mol) N-Isopropyliden-2,6-dimethylanilin getropft wobei die Temperatur bei 0°C gehalten wird. Anschließend wird noch 3 Stunden bei Raumtemperatur nachgerührt. Man filtriert vom ausgefallenen Niederschlag (N-Isopropyliden-2,6-diethylamin-hydrochlorid) ab, wäscht das Filtrat zunächst mehrfach mit wäßriger Bicarbonatlösung und dann mit Wasser. Die organische Phase wird über Natriumsulfat getrocknet und im Vakuum eingedampft. Der Rückstand wird im Vakuum fraktioniert destilliert. Man erhält 180,6g (76 % der Theorie) N-(1-Methylvinyl)-N-(2,6-dimethylphenyl)-chloracetamid als farbloses Oel vom Siedepunkt 98-106°C/0,15 mbar.

**Biespiel 6**

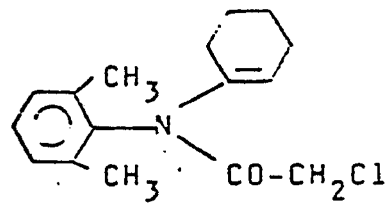

Zu einer Lösung von 113g (1 Mol) Chloracetylchlorid in 200 ml Toluol werden unter Rühren bei 0° C bis 10° C 209 g (1 Mol) N-Cyclohexyliden-2,6-dimethylanilin getropft. Man läßt 3 Stunden bei Raumtemperatur nachrühren und erhitzt dann so lange unter Rückfluß, bis kein Chlorwasserstoff mehr entweicht. Danach wird die Lösung im Vakuum eingeengt. Man erhält 21,9g eines festen Rückstandes, der aus Ligroin fraktioniert umkristallisiert wird.

Man erhält 18 g (65 % der Theorie) N-(1-Cyclohexenyl)-N-(2,6-dimethylphenyl)-chloracetamid in Form gelb gefärbter Kristalle vom Schmelzpunkt 78° C.

In analoger Weise werden die in der folgenden Tabelle aufgeführten Verbindungen der Formel (I) erhalten:

**Tabelle 1**

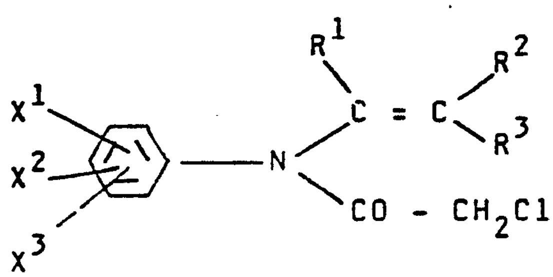

(I)

| Bsp. Nr. | $X^1$ | $X^2$ | $X^3$ | $-CR^1=CR^2R^3$ | Physikalische Konstante |
|---|---|---|---|---|---|
| 7 | $2-CH_3$ | $6-CH_3$ | H | $-CH=CH_2$ | Fp:85°C |
| 8 | $2-C_2H_5$ | $6-CH_3$ | H | $-CH=CH_2$ | Kp:90°C/mbar |
| 9 | $2-C_2H_5$ | $6-CH_3$ | H | $-C(CH_3)=CH_2$ | Kp:93-97°C/ 0,1 mbar |
| 10 | $2-CH_3$ | $6-CH_3$ | H | 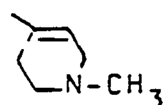 | Fp: 92°C |
| 11 | $2-CH_3$ | $6-CH_3$ | H | 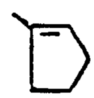 | Öl |
| 12 | $2-CH_3$ | $6-CH_3$ | H | $-CH=CH-CO-CH_3$ | Fp:147°C |
| 13 | 2-Cl | $6-CH_3$ | H | 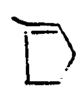 | Öl |
| 14 | H | H | H | 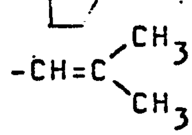 | Fp:85°C |
| 15 | $2-CH_3$ | $6-CH_3$ | H | 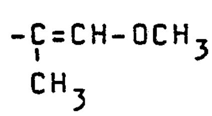 | Fp:110°C |

**T a b e l l e 1** (Fortsetzung)

| Bsp. Nr. | $X^1$ | $X^2$ | $X^3$ | $-CR^1=CR^2R^3$ | Physikalische Konstante |
|---|---|---|---|---|---|
| 16 | $2-CH_3$ | $6-CH_3$ | H | $-CH=CH-C_2H_5$ | Fp: 57°C |
| 17 | $2-CH_3$ | $6-CH_3$ | H | $-CH=C(CH_3)_2$ | Öl |
| 18 | $2-CH_3$ | $6-CH_3$ | H | $-C(C_3H_7-i)=CH_2$ | Öl |
| 19 | $2-CH_3$ | $6-CH_3$ | H | $-C(C_4H_9-t)=CH_2$ | Öl |
| 20 | $2-CH_3$ | $6-CH_3$ | H | 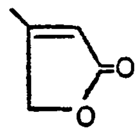 | Fp: 176°C |

**Herstellung von Ausgangsprodukten der Formel (II)**

**Beispiel (II-1)**

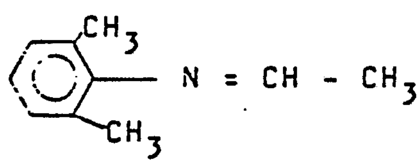

121g (1 Mol) 2,6-Dimethylanilin in 200 ml Methylenchlorid werden mit 480g (3 Mol) Natriumsulfat und 3 ml Dimethylbenzylamin versetzt. Dazu werden bei 0°C 88g (2 Mol) Acetaldehyd zugegeben. Man läßt 4 Stunden bei 0°C rühren, filtriert und engt das Filtrat im Vakuum bei maximal 20°C Badtemperatur ein. Man erhält 140 g (95,2 % der Theorie) N-Ethyliden-2,6-dimethylanilin als rötliches Öl.

**Beispiel (II-2)**

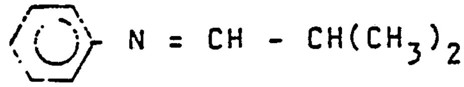

Zu einer Lösung von 92 g (1 Mol) Anilin, 25o ml Methylenchlorid, 2 ml Dimethylbenzylamin und 150 g wasserfreiem Magnesiumsulfat tropft man bei 0°C unter Rühren innerhalb 1 Stunde 144g (2 Mol) Isobutyraldehyd zu. Man läßt 5 Stunden bei 0 bis 10°C nachrühren, filtriert und wäscht den Rückstand mit 200 ml Methylenchlorid nach. Die vereinigten organischen Phasen werden im Vakuum bei 30 bis 40°C vom Lösungsmittel befreit. Man erhält 155g rohes N-Isobutyliden-anilin, das direkt weiter umgesetzt wird.

**Beispiel (II-3)**

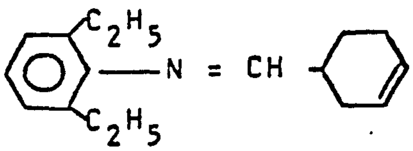

447 g -(3 Mol) 2,6-Diethylanilin und 327g (3 Mol) 3-Cyclo-hexen-1-carboxaldehyd werden in 1000 ml Toluol 9 Stunden am Wasserabscheider unter Rückfluß erhitzt, bis kein Wasser mehr abgeschieden wird. Danach wird das Reaktionsgemisch durch Abdestillieren des Lösungsmittels im Vakuum eingeengt, und der Rückstand im Vakuum destilliert. Man erhält 615 g (85 % der Theorie) N-(3-Cyclo-hexenyl-methyliden)-2,6-diethylanilin vom Siedepunkt 110-12° C/0,1 mbar.

**Beispiel (II-4)**

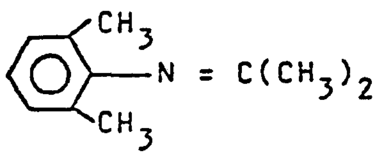

Ein Gemisch aus 121 g Dimethylanilin und 156 g (1,5 Mol) 2,2-Dimethoxypropan werden mit 0,1 g p-Toluolsulfonsäure versetzt und 30 Minuten unter Rückfluß erhitzt. Anschließend wird das entstandene Methanol abdestilliert. Zur Vervollständigung der Reaktion werden nochmals 52 g (0,5 Mol) 2,2-Dimethoxypropan und 0,05 g p-Toluolsulfonsäure zugesetzt und das Reaktionsgemisch destilliert bis der Siedepunkt des 2,2-Dimethoxypropans (83° C) erreicht ist. Die verbleibende Lösung wird im Vakuum eingedampft. Man erhält 157g (97 % der Theorie) N-Isopropyliden-2,6-dimethylanilin in Form eines farblosen Öls.

**Beispiel (II-5)**

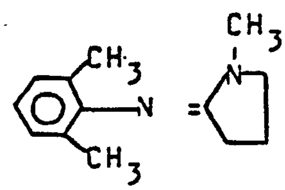

Zu einer Lösung von 99,15g (1 Mol) N-Methylpyrrolidon in 375 ml Toluol tropft man bei 20-25° C 76,7g (0,5 Mol) Phosphoroxichlorid. Man rührt 2 Stunden nach und tropft anschließend eine Lösung von 60,6 g (0,5 Mol) 2,6-Dimethyl-anilin in 125 ml Toluol bei 20-30° C zu. Das Reaktionsgemisch wird anschließend 4 Stunden auf 70° C erhitzt. Nach dem Abkühlen wird die Toluolphase abgetrennt, der verbleibende ölige Rückstand in Wasser aufgenommen, mit wäßriger Natronlauge auf pH 13 gebracht und mehrfach mit Chloroform extrahiert. Die vereinigten Extrakte werden über Natriumsulfat getrocknet und eingeengt. Der dabei erhaltene Rückstand wird im Vakuum destilliert.

Man erhält 98,7g (40 % der Theorie) N-(N'-Methyl-2-pyrrolidenyl)-2,6-dimethylanilin vom Siedepunkt 87-89° C/0,3 mbar.

In analoger Weise werden die in der folgenden Tabelle 2 aufgeführten Ausgangsstoffe der Formel (II) erhalten:

**Tabelle 2**

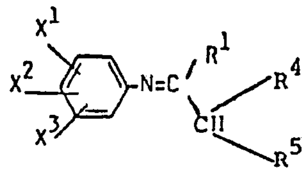

(II)

| Bsp. Nr. | $X^1$ | $X^2$ | $X^3$ | $R^1$ | $R^4$ | $R^5$ | Physikalische Konstante |
|---|---|---|---|---|---|---|---|
| II-6 | $2-C_2H_5$ | $6-CH_3$ | H | H | H | H | Öl |
| II-7 | $2-C_2H_5$ | $6-C_2H_5$ | H | H | H | H | Öl |
| II-8 | $2-C_2H_5$ | $6-CH_3$ | H | $CH_3$ | H | H | Öl |
| II-9 | $2-CH_3$ | $6-CH_3$ | H | H | $CH_3$ | $CH_3$ | Kp: 108-110°C/20mbar |
| II-10 | $2-CH_3$ | $6-C_2H_5$ | H | H | $CH_3$ | $CH_3$ | Kp: 112-115°C/20mbar |
| II-11 | $2-C_2H_5$ | $6-C_2H_5$ | H | H | $CH_3$ | $CH_3$ | Kp: 123-125°C/20mbar |
| II-12 | $2-CH_3$ | $6-CH_3$ | H | H | 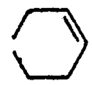 | | Kp: 86°C/0,2mbar |
| II-13 | $2-C_2H_5$ | $6-C_2H_5$ | H | H | 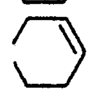 | | Kp: 110-112°C/0,15mbar |
| II-14 | $2-CH_3$ | $6-C_2H_5$ | H | H | 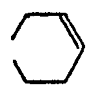 | | Kp: 107-110°C/0,5mbar |
| II-15 | $2-CH_3$ | $6-CH_3$ | H | H | 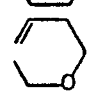 | | Kp: 111°C/0,3mbar |
| II-16 | $2-CH_3$ | $6-CH_3$ | H | 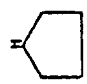 | | H | Kp: 133-140°C/20mbar |
| II-17 | $2-CH_3$ | $6-C_2H_5$ | H | 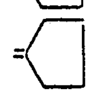 | | H | Kp: 125-135°C/20mbar |
| II-18 | $2-CH_3$ | $6-CH_3$ | H | 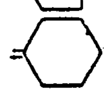 | | H | Kp: 150-152°C/11mbar |
| II-19 | $2-CH_3$ | $6-CH_3$ | H | 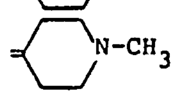 | | H | Kp: 95°C/0,3mbar |
| II-20 | $2-CH_3$ | $6-CH_3$ | H | H | H | $-CO-CH_3$ | Kp: 94°C/0,15mbar |
| II-21 | $2-CH_3$ | $6-CH_3$ | H | $CH_3$ | $OCH_3$ | $OCH_3$ | Kp: 93°C/0,6mbar |
| II-22 | $2-CH_3$ | $6-CH_3$ | H | $CH_3$ | H | $OCH_3$ | Kp: 70°C/0,2mbar |
| II-23 | 2-Cl | $6-CH_3$ | H | | 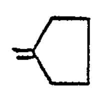 | H | Kp: 142°C/16mbar |
| II-24 | $2-CH_3$ | $6-CH_3$ | H | H | H | $-COOC_2H_5$ | Kp: 90-92°C/20mbar |
| II-25 | $2-CH_3$ | $6-CH_3$ | H | 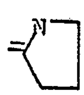 | | H | Öl |

**Tabelle 2** (Fortsetzung)

| Bsp. Nr. | $X^1$ | $X^2$ | $X^3$ | $R^1$ | $R^4$ | $R^5$ | Physikalische Konstante |
|---|---|---|---|---|---|---|---|
| II-26 | 2-$CH_3$ | 6-$CH_3$ | H | H | H | $C_2H_5$ | Kp: 65°C/0,2 mbar |
| II-27 | 2-$CH_3$ | 6-$CH_3$ | H | $CH_3$ | $CH_3$ | $CH_3$ | Kp: 64°C/0,2 mbar |
| II-28 | 2-$CH_3$ | 6-$CH_3$ | H | 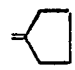 | | -$COCH_3$ | Fp: 55°C |
| II-29 | 2-$CH_3$ | 6-$CH_3$ | H | 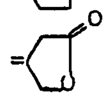 | | H | Fp: 199°C |
| II-30 | 2-$CH_3$ | 4-$CH_3$ | 6-$CH_3$ | $CH_3$ | H | H | Öl |
| II-31 | 3-Cl | 5-Cl | H | 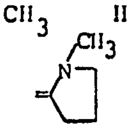 | | H | Öl |
| II-32 | 2-$CH_3$ | 6-$CH_3$ | H | -$C(CH_3)_3$ | H | H | Kp: 58°C/0,1 mbar |

**Anwendungsbeispiele:**

In dem folgenden Anwendungsbeispiel A wird die nachstehend angegebene Verbindung als Vergleichssubstanz eingesetzt:
(A) =

(A) = 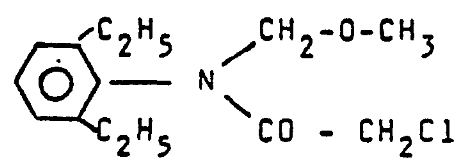

2,6-Diethyl-N-methoxymethyl-chloracet-anilid
(bekannt aus US-PS 3 442 945)

**Beispiel A**

Pre-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe (1), (6), (8), (9) und (10) zeigen in diesem Test bessere selektive Wirksamkeit als die aus dem Stand der Technik bekannte Verbindung (A).

**Beispiel 8**

Wuchshemmung bei Gras (Festuca pratensis)

Lösungsmittel: 30 Gewichtsteile Dimethylformamid

Emulgator : 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Sillstand des Wachstums und 0 % ein Wachstum entsprechend dem der Kontrollpflanzen.

Der erfindungsgemäße Wirkstoff (8) zeigt in diesem Test eine sehr starke Wuchshemmung.

**Patentansprüche**

1. N-(1-Alkenyl)-chloressigsäureanilide der Formel

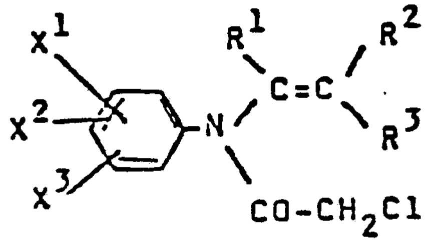

(I)

in welcher

$R^1$ für Wasserstoff oder geradkettiges oder verzweigtes Alkyl mit 1 bis 3 Kohlenstoffatomen steht,

$R^2$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl steht,

$R^3$ für Wasserstoff, geradkettiges oder verzweigtes Alkyl mit 1 bis 4 Kohlenstoffatomen, Alkoxy mit 1 bis 4 Kohlenstoffatomen, Alkoxyalkyl mit 1 bis 4 Kohlenstoffatomen im Alkylteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylakyl mit 1 bis 4 Kohlenstoffatomen im Alkoxyteil und 1 bis 4 Kohlenstoffatomen im Alkylteil steht, oder

$R^1$ und $R^2$ gemenisam mit der C=C-Doppelbindung für einen gegebenenfalls ein- oder mehrfach substitutierten, einfach oder mehrfach ungesättigten, 5- oder 6-gliedrigen Ring stehen, der ein oder zwei Heteroatome und/oder eine Carbonyl-Gruppe enthalten kann, wobei als Substituenten beispielsweise genannt seien: Alkyl mit 1 bis 4 Kohlenstoffatomen, Halogen und Hydroxy;

oder

$R^2$ und $R^3$ gemeinsann mit dem angrenzenden Kohlenstoffatom für einen gegebenenfalls ein- oder mehrfach substituierten, gesättigten oder ungesättigten, 5- oder 6-gliedrigen Ring stehen, der ein oder zwei Heteroatome enthalten kann, wobei als Substituenten beispielsweise genannt seien: Alkyl mit 1 bis 4 Kohlenstoffatomen, Halogen und Hydroxy;

$X^1$ für Wasserstoff, geradkettiges oder verzweigtes Alkyl mit 1 bis 4 Kohlenstoffatomen, Fluor, Chlor oder Brom steht,

$X^2$ für Wasserstoff, geradkettiges oder verzweigtes Alkyl mit 1 bis 4 Kohlenstoffatomen, Fluor, Chlor oder Brom steht, und

$X^3$ für Wasserstoff und geradkettiges Alkyl mit 1 bis 4 Kohlenstoffatomen, Fluor, Chlor oder Brom steht.

2. N-1-Alkenyl)-chloressigsäureanilid der Formel

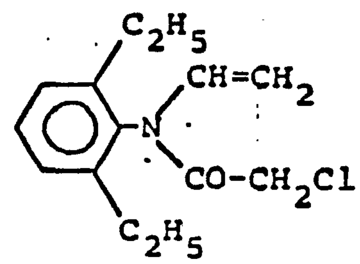

3. N-(1-Alkenyl)-chloressigsäureanilid der Formel

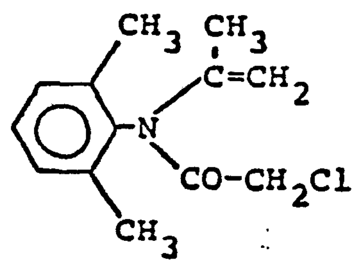

4. N-(1-Alkenyl)-chloressigsäureanilid der Formel

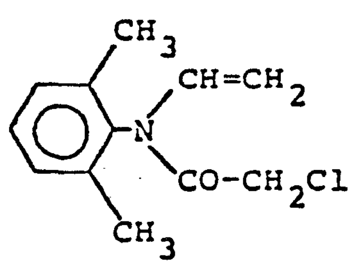

5. Verfahren zur Herstellung von N-(1-Alkenyl)-chloressigsäureaniliden der Formel

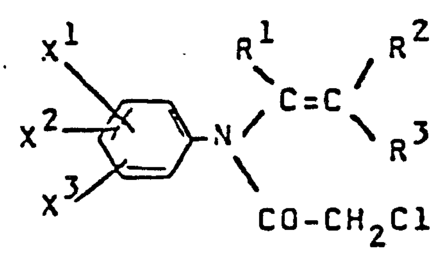

(I)

in welcher

$R^1$ für Wassersstoff oder geradkettiges oder verzweigtes Alkyl mit 1 bis 4 Kohlenstoffatomen steht,

$R^2$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl steht,

$R^3$ für Wasserstoff, geradkettiges oder verzweigtes Alkyl mit 1 bis 4 Kohlenstoffatomen, Alkoxy mit 1 bis 4

Kohlenstoffatomen, Alkoxyalkyl mit 1 bis 4 Kohlenstoffatomen im Alkylteil und 1 bis 4 Kohlenstoffatomen im Alkoxyteil,

Alkylcarbonyl mit 1 bis 4 Kohlenstoffatomen im Alkylteil, Alkylcarbonylalkyl mit 1 bis 1 Kohlenstoffatomen in jedem Alkylteil, Alkoxycarbonyl mit 1 bis 1 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkylteil steht, oder

$R^1$ und $R^2$ gemeinsam mit der C=C-Doppelbindung für einen gegebenfalls ein- oder mehrfach substitutierten, einfach oder mehrfach ungesättigten, 5- oder 6-gliedrigen Ring stehen, der ein oder zwei Heteroatome und/oder eine Carbonyl Gruppe enthalten kann, wobei als Substituenten beispielsweise genannt seien: Alkyl mit 1 bis 4 Kohlenstoffatomen, Halogen und Hydroxy;

$R^2$ und $R^3$ gemeinsam mit dem angrenzenden Kohlenstoffatom für einen gegebenenfalls ein- oder mehrfach substituierten, gesättigten oder ungesättigten, 5- oder 6-gliedrigen Ring stehen, der ein oder zwei Heteroatome enthalten kann, wobei als Substituenten beispielsweise genannt seien: Alkyl mit 1 bis 4 Kohlenstoffatomen, Halogen und Hydroxy;

$X^1$ für Wasserstoff, geradkettiges oder verzweigtes Alkyl mit 1 bis 4 Kohlenstoffatomen, Fluor, Chlor oder Brom steht,

$X^2$ für Wasserstoff, geradkettiges oder verzweigtes Alkyl mit 1 bis 4 Kohlenstoffatomen, Fluor, Chlor oder Brom steht, und

$X^3$ für Wasserstoff und geradkettiges Alkyl mit 1 bis 4 Kohlenstoffatomen, Fluor, Chlor oder Brom steht,

dadurch gekennzeichnet, daß man Azine der Formel

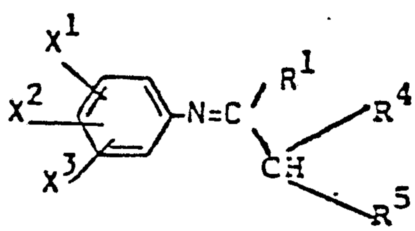

(II)

in welcher

$R^1$, $X^1$, $X^2$ und $X^3$ die oben angegebene Bedeutung haben,

$R^4$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl steht,

$R^5$ für Wasserstoff, geradkettiges oder verzweigtes Alkyl mit 1 bis 4 Kohlenstoffatomen, Alkoxy mit 1 bis 4 Kohlenstoffatomen, Alkoxyalkyl mit 1 bis 4 Kohlenstoffatomen im Alkylteil und 1 bis 4 Kohlenstoffatomen im Alkoxyteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkylteil steht, oder

$R^1$ und $R^4$ gemeinsam mit der angrenzenden C-C-Bindung für einen gegebenenfalls ein- oder mehrfach substitutierten, einfach oder mehrfach ungesättigten, 5- oder 6-gliedrigen Ring stehen, der ein oder zwei Heteroatome und/oder eine Carbonyl-Gruppe enthalten kann, wobei als Substituenten beispielsweise genannt seien: Alkyl mit 1 bis 4 Kohlenstoffatomen, Halogen und Hydroxy;

oder

$R^4$ und $R^5$ gemeinsam mit dem angrenzenden Kohlenstoffatom für einen gegebenenfalls ein- oder mehrfach substituierten, gesättigten oder ungesättigten, 5- oder 6-gliedrigen Ring stehen, der ein oder zwei Heteroatome enthalten kann, wobei als Substituenten beispielsweise genannt seien: Alkyl mit 1 bis 4 Kohlenstoffatomen, Halogen und Hydroxy;

mit Chloracethalogeniden der Formel

$Hal-CO-CH_2-Cl$ (III)

in welcher

Hal für Chlor oder Brom steht,

gegebenenfalls in Gegenwart eines Verdünnungsmittels umsetzt und die entstehenden Chloracetanilide der Formel

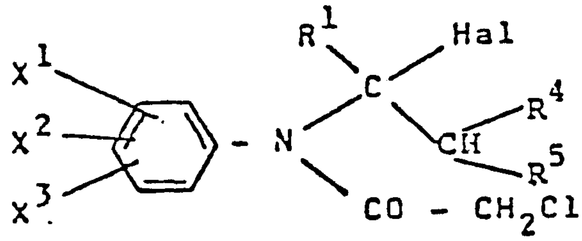

(IV)

in welcher $R^1$, $R^4$, $R^5$, $X^1$, $X^2$, $X^3$ und Hal die oben angegebene Bedeutung haben, ohne vorherige Isolierung, gegebenenfalls in Gegenwart einer Base, der Halogenwasserstoffeliminierung unterwirft.

6. Herbizide und pflanzenwuchsregulierende Mittel, gekennzeichnet durch einen Gehalt an mindestens einem N-(1-Alkenyl)-chloressigsäureanilid der Formel (I).

7. Verfahren zur Bekämpfung von Unkräutern bzw. zur Regulierung des Pflanzenwachstums, dadurch gekennzeichnet, daß man N-(1-Alkenyl)-chloressigsäureanilide der Formel (I) auf die Unkräuter bzw. die zu regulierenden Pflanzen und/oder deren Lebensraum ausbringt.

8. Verwendung von N-(1-Alkenyl)-chloressigsäureaniliden der Formel (I) zur Bekämpfung von Unkräutern bzw. zur Regulierung des Pflanzenwachstums.

9. Verfahren zur Herstellung von herbiziden bzw. pflanzenwichsregulierenden Mitteln, dadurch gekennzeichnet, daß man N-(1-Alkenyl)-chloressigsäureanilide der Formel (I) mit Streckmitteln und/oder oberflächenaktiven Stoffen vermischt.

## Claims

1. N-(1-alkenyl)-chloroacetic acid anilides of the formula

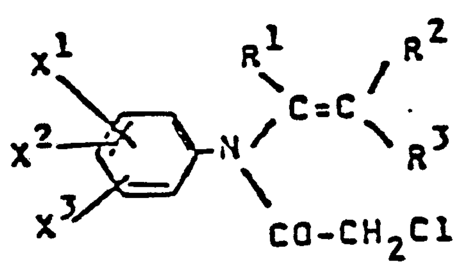

(I)

in which

$R^1$ represents hydrogen or straight-chain or branched alkyl with 1 to 4 carbon atoms,

$R^2$ represents hydrogen, straight-chain or branched alkyl with 1 to 4 carbon atoms, alkoxy with 1 to 4 carbon atoms, hydroxyalkyl with 1 to 4 carbon atoms, alkoxyalkyl with 1 to 4 carbon atoms in the alkyl part and 1 to 4 carbon atoms in the alkoxy part or benzyl,

$R^3$ represents hydrogen, straight-chain or branched alkyl with 1 to 4 carbon atoms, alkoxy with 1 to 4 carbon atoms, alkoxyalkyl with 1 to 4 carbon atoms in the alkyl part and 1 to 4 carbon atoms in the alkoxy part, alkylcarbonyl with 1 to 4 carbon atoms in the alkyl part, alkylcarbonylalkyl with 1 to 4 carbon atoms in each alkyl part, alkoxycarbonyl with 1 to 4 carbon atoms in the alkoxy part, alkoxycarbonylalkyl with 1 to 4 carbon atoms in the alkoxy part and 1 to 4 carbon atoms in the alkyl part, or

$R^1$ and $R^2$, together with the C=C double bond, represent an optionally mono- or polysubstituted, mono- or polyunsaturated 5- or 6-membered ring which can contain one or two heteroatoms and/or a carbonyl group, the following being mentioned as examples of substituents: alkyl with 1 to 4 carbon atoms, halogen and hydroxyl;

or

$R^2$ and $R^3$, together with the adjacent carbon atom, represent an optionally mono- or polysubstituted, saturated or unsaturated 5- or 6-membered ring which can contain one or two heteroatoms, the following being mentioned as examples of substituents: alkyl with 1 to 4 carbon atoms, halogen and hydroxyl;

$X^1$ represents hydrogen, straight-chain or branched alkyl with 1 to 4 carbon atoms, fluorine, chlorine or bromine,

$X^2$ represents hydrogen, straight-chain or branched alkyl with 1 to 4 carbon atoms, fluorine, chlorine or bromine, and

$X^3$ represents hydrogen and straight-chain alkyl with 1 to 4 carbon atoms, fluorine, chlorine or bromine.

2. N-(1-alkenyl)-chloroacetic acid anilide of the formula

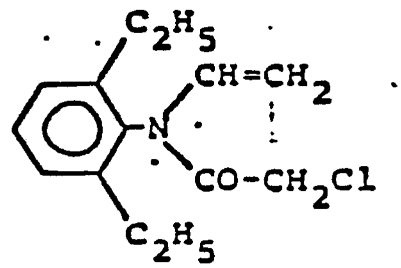

3. N-(1-alkenyl)-chloroacetic acid anilide of the formula

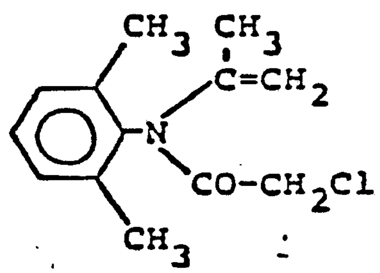

4. N-(1-alkenyl)-chloroacetic acid anilide of the formula

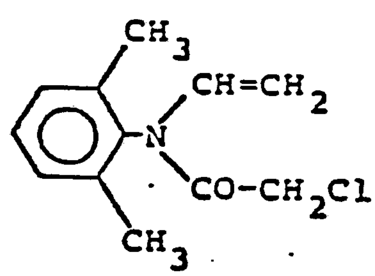

5. Process for the preparation of N-(1-alkenyl)-chloroacetic acid anilides of the formula

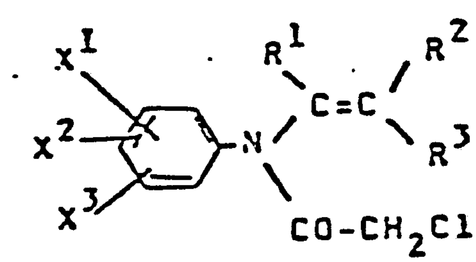

(I)

in which

$R^1$ represents hydrogen or straight-chain or branched alkyl with 1 to 4 carbon atoms,

$R^2$ represents hydrogen, straight-chain or branched alkyl with 1 to 4 carbon atoms, alkoxy with 1 to 4 carbon atoms, hydroxyalkyl with 1 to 4 carbon atoms, alkoxyalkyl with 1 to 4 carbon atoms in the alkyl part and 1 to 4 carbon atoms in the alkoxy part or benzyl,

$R^3$ represents hydrogen, straight-chain or branched alkyl with 1 to 4 carbon atoms, alkoxy with 1 to 4 carbon atoms, alkoxyalkyl with 1 to 4 carbon atoms in the alkyl part and 1 to 4 carbon atoms in the alkoxy part, alkylcarbonyl with 1 to 4 carbon atoms in the alkyl part, alkylcarbonylalkyl with 1 to 4 carbon atoms in each alkyl part, alkoxycarbonyl with 1 to 4 carbon atoms in the alkoxy part, alkoxycarbonylalkyl with 1 to 4 carbon atoms in the alkoxy part and 1 to 4 carbon atoms in the alkyl part, or

$R^1$ and $R^2$, together with the C=C double bond represent an optionally mono- or polysubstituted, mono- or polyunsaturated 5- or 6-membered ring which can contain one or two heteroatoms and/or a carbonyl group, the following being mentioned as examples of substituents: alkyl with 1 to 4 carbon atoms, halogen and hydroxyl;

or

$R^2$ and $R^3$, together with the adjacent carbon atom, represent an optionally mono- or polysubstituted, saturated or unsaturated 5- or 6-membered ring which can contain one or two heteroatoms, the following being mentioned as examples of substituents: alkyl with 1 to 4 carbon atoms, halogen and hydroxyl,

$X^1$ represents hydrogen, straight-chain or branched alkyl with 1 to 4 carbon atoms, fluorine, chlorine or bromine,

$X^2$ represents hydrogen, straight-chain or branched alkyl with 1 to 4 carbon atoms, fluorine, chlorine or bromine, and

$X^3$ represents hydrogen and straight-chain alkyl with 1 to 4 carbon atoms, fluorine, chlorine or bromine,

characterised in that azines of the formula

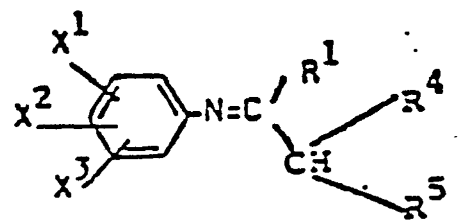

(II)

in which

$R^1$, $X^1$, $X^2$ and $X^3$ have the abovementioned meaning,

$R^4$ represents hydrogen, straight-chain or branched alkyl with 1 to 4 carbon atoms, alkoxy with 1 to 4 carbon atoms, hydroxyalkyl with 1 to 4 carbon atoms, alkoxyalkyl with 1 to 4 carbon atoms in the alkyl part and 1 to 4 carbon atoms in the alkoxy part or benzyl,

$R^5$ represents hydrogen, straight-chain or branched alkyl with 1 to 4 carbon atoms, alkoxy with 1 to 4 carbon atoms, alkoxyalkyl with 1 to 4 carbon atoms in the alkyl part and 1 to 4 carbon atoms in the alkoxy part, alkylcarbonyl with 1 to 4 carbon atoms in the alkyl part, alkylcarbonylalkyl with 1 to 4 carbon atoms in each alkyl part, alkoxycarbonyl with 1 to 4 carbon atoms in the alkoxy part, alkoxycarbonylalkyl with 1 to 4 carbon atoms in the alkoxy part and 1 to 4 carbon atoms in the alkyl part, or

$R^1$ and $R^4$, together with the adjacent C-C bond, represent an optionally mono- or polysubstituted, mono- or polyunsaturated 5- or 6-membered ring which can contain one or two heteroatoms and/or a carbonyl group, the following being mentioned as examples of substituents: alkyl with 1 to 4 carbon atoms, halogen and hydroxyl;

or

$R^4$ and $R^5$, together with the adjacent carbon atom, represent an optionally mono- or polysubstituted, saturated or unsaturated 5- or 5-membered ring which can contain one or two heteroatoms, the following being mentioned as examples of substituents: alkyl with 1 to 4 carbon atoms, halogen and hydroxyl;

are reacted with chloroacetyl halides of the formula

$Hal-CO-CH_2-CL$ (III)

in which

Hal represents chlorine or bromine,

if appropriate in the presence of a diluent and the resulting chloroacetanilides of the formula

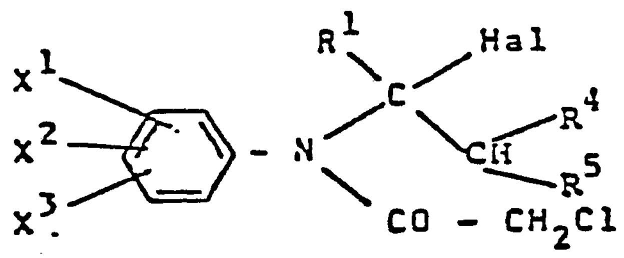

(IV)

in which

$R^1$, $R^4$, $R^5$, $X^1$, $X^2$, $X^3$ and Hal have the abovementioned meaning,

are subjected, without prior isolation, to elimination of hydrogen halide, if appropriate in the presence of a base.

6. Herbicidal and plant-growth-regulating agents, characterised in that they contain at least one N-(1-alkenyl)-chloroacetic acid anilide of the formula (I).

7. Method of combating weeds or of regulating plant growth, characterised in that N-(1-alkenyl)-chloroacetic acid anilides of the formula (I) are applied to the weeds or the plants to be regulated and/or their environment.

8. Use of N-(1-alkenyl)-chloroacetic acid anilides of the formula (I) for combating weeds or for regulating plant growth.

9. Process for the preparation of herbicidal or plant-growth-regulating agents, characterised in that N-(1-alkenyl)chloroacetic acid anilides of the formula (I) are mixed with extenders and/or surface-active substances.

**Revendications**

1. N-(1-alcényl)-chloracétanilides de formule:

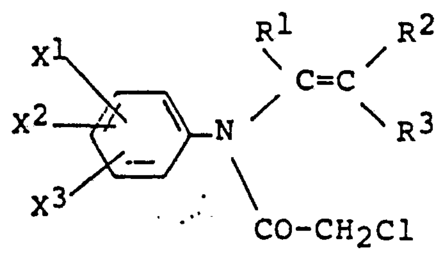

(I)

dans laquelle

$R_1$ représente de l'hydrogène ou un alcoyle à chaîne droite ou ramifiée ayant 1 à 4 atomes de carbone,

$R^2$ de l'hydrogène, un alcoyle à chaîne droite ou ramifiée ayant 1 à 4 atomes de carbone, un alcoxy ayant 1 à 4 atomes de carbone, un hydroxyalcoyle ayant 1 à 4 atomes de carbone, un alcoxyalcoyle ayant 1 à 4 atomes de carbone dans la portion alcoyle et 1 à 4 atomes de carbone dans la portion alcoxy, ou un benzyle,

$R^3$ de l'hydrogène, un alcoyle à chaîne droite ou ramifiée ayant 1 à 4 atomes de carbone, un alcoxy ayant 1 à 4 atomes de carbone, un alcoxyalcoyle ayant 1 à 4 atomes de carbone dans la portion alcoyle et 1 à 4 atomes de carbone dans la portion alcoxy, un alcoylcarbonyle ayant 1 à 4 atomes de carbone dans la portion alcoyle, un alcoylcarbonylalcoyle ayant 1 à 4 atomes de carbone dans chaque portion alcoyle, un alcoxycarbonyle ayant 1 à 4 atomes de carbone dans la portion alcoxy, un alcoxycarbonylalcoyle ayant 1 à 4 atomes de carbone dans la portion alcoxy et 1 à 4 atomes de carbone dans la portion alcoyle, ou bien

$R^1$ et $R^2$, conjointement avec la double liaison C=C, représentent un noyau à 5 ou 6 chaînons mono- ou polyinsaturé, éventuellement mono- ou polysubstitué, qui peut contenir un ou deux hétéroatomes et/ou un groupe carbonyle, en citant par exemple comme substituants: alcoyle ayant 1 à 4 atomes de carbone, halogène et hydroxy,

ou bien

$R^2$ et $R^3$, conjointement avec l'atome de carbone voisin, représentent un noyau à 5 ou 6 chaînons, saturé ou non saturé, éventuellement mono- ou polysubstitué, qui peut contenir un ou deux hétéroatomes, en citant par exemple comme substituants: alcoyle ayant 1 à 4 atomes de carbone, halogène et hydroxy,

$X^1$ représente de l'hydrogène, un alcoyle à chaîne droite ou ramifiée ayant 1 à 4 atomes de carbone, du fluor, du chlore ou du brome,

$X^2$ de l'hydrogène, un alcoyle à chaîne droite ou ramifiée ayant 1 à 4 atomes de carbone, du fluor, du chlore ou du brome et

$X^3$ de l'hydrogène et un alcoyle à chaîne droite ayant 1 à 4 atomes de carbone, du fluor, du chlore ou du brome.

2. N-(1-alcényl)-chloracétanilide de formule:

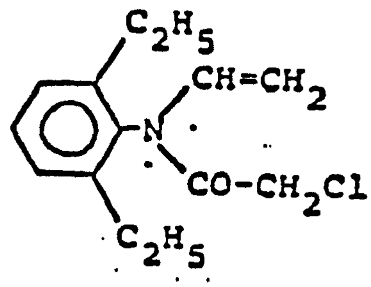

3. N-(1-alcényl)-chloracétanilide de formule:

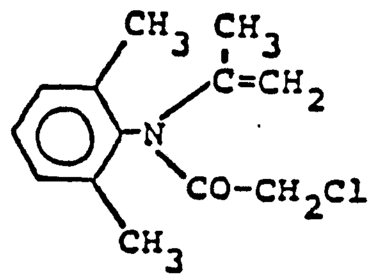

4. N-(1-alcényl)-chloracétanilide de formule:

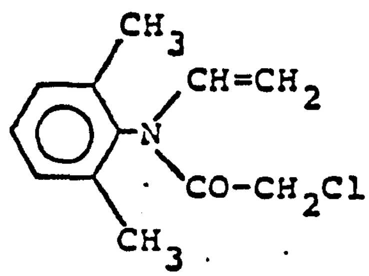

5. Procédé de fabrication de N-(1-alcényl)-chloracétanilides de formule:

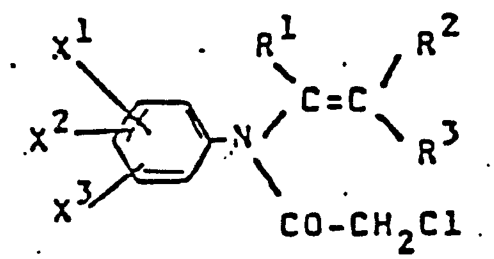

(I)

dans laquelle

$R^1$ représente de l'hydrogène ou un alcoyle à chaîne droite ou ramifiée ayant 1 à 4 atomes de carbone,

$R^2$ de l'hydrogène, un alcoyle à chaîne droite ou ramifiée ayant 1 à 4 atomes de carbone, un alcoxy ayant 1 à

4 atomes de carbone, un hydroxyalcoyle ayant 1 à 4 atomes de carbone, un alcoxyalcoyle ayant 1 à 4 atomes de carbone dans la portion alcoyle et 1 à 4 atomes de carbone dans la portion alcoxy, ou un benzyle,

$R^3$ de l'hydrogène, un alcoyle à chaîne droite ou ramifiée ayant 1 à 4 atomes de carbone, un alcoxy ayant 1 à 4 atomes de carbone, un alcoxyalcoyle ayant 1 à 4 atomes de carbone dans la portion alcoyle et 1 à 4 atomes de carbone dans la portion alcoxy, un alcoylcarbonyle ayant 1 à 4 atomes de carbone dans la portion alcoyle, un alcoylcarbonylalcoyle ayant 1 à 4 atomes de carbone dans chaque portion alcoyle, un alcoxycarbonyle ayant 1 à 4 atomes de carbone dans la portion alcoxy, un alcoxycarbonylalcoyle ayant 1 à 4 atomes de carbone dans la portion alcoxy et 1 à 4 atomes de carbone dans la portion alcoyle, ou bien

$R^1$ et $R^2$, conjointement avec la double liaison C=C, représentent un noyau à 5 ou 6 chaînons, mono- ou polyinsaturé, éventuellement mono- ou polysubstitué, qui peut contenir un ou deux hétéroatomes et/ou un groupe carbonyle, en citant en exemple comme substituants: alcoyle ayant 1 à 4 atomes de carbone, halogène et hydroxy,

ou bien

$R^2$ et $R^3$, conjointement avec l'atome de carbone voisin, représentent un noyau à 5 ou 6 chaînons, saturé ou insaturé, éventuellement mono- ou polysubstitué, qui peut contenir un ou deux hétéroatomes, en citant par exemple comme substituants: alcoyle ayant 1 à 4 atomes de carbone, halogène et hydroxy,

$X^1$ représente de l'hydrogène, un alcoyle à chaîne droite ou ramifiée ayant 1 à 4 atomes de carbone, du fluor, du chlore ou du brome,

$X^2$ de l'hydrogène, un alcoyle à chaîne droite ou ramifiée ayant 1 à 4 atomes de carbone, du fluor, du chlore ou du brome et :

$X^3$ de l'hydrogène et un alcoyle à chaîne droite ayant 1 à 4 atomes de carbone, du fluor, du chlore ou du brome,

caractérisé en ce qu'on fait réagir des azines de formule:

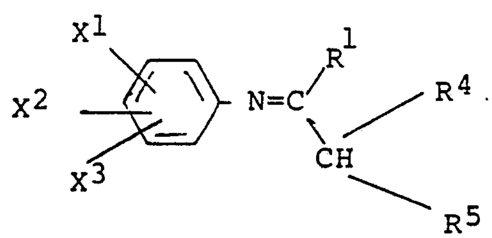

(II)

dans laquelle

$R^1$, $X^1$, $X^2$ et $X^3$ ont la signification indiquée plus haut,

$R^4$ représente de l'hydrogène, un alcoyle à chaîne droite ou ramifiée ayant 1 à 4 atomes de carbone, un alcoxy ayant 1 à 4 atomes de carbone, un hydroxyalcoyle ayant 1 à 4 atomes de carbone, un alcoxyalcoyle ayant 1 à 4 atomes de carbone dans la portion alcoyle et 1 à 4 atomes de carbone dans la portion alcoxy, ou un benzyle,

$R^5$ de l'hydrogène, un alcoyle à chaîne droite ou ramifiée ayant 1 à 4 atomes de carbone, un alcoxy ayant 1 à 4 atomes de carbone, un alcoxyalcoyle ayant 1 à 4 atomes de carbone dans la portion alcoyle et 1 à 4 atomes de carbone dans la portion alcoxy, un alcoxycarbonyle ayant 1 à 4 atomes de carbone dans la portion alcoyle, un alcoylcarbonylalcoyle ayant 1 à 4 atomes de carbone dans chaque portion alcoyle, un alcoxycarbonyle ayant 1 à 4 atomes de carbone dans la portion alcoxy, un alcoxycarbonylalcoyle ayant 1 à 4 atomes de carbone dans la portion alcoxy et 1 à 4 atomes de carbone dans la portion alcoyle, ou bien

$R^1$ et $R^4$, conjointement avec la liaison voisine C-C, représentent un noyau à 5 ou 6 chaînons, mono- ou polyinsaturé, éventuellement mono- ou polysubstitué, qui peut contenir un ou deux hétéroatomes et/ou un groupe carbonyle, en citant par exemple comme substituants: alcoyle ayant 1 à 4 atomes de carbone, halogène et hydroxy,

ou bien

$R^4$ et $R^5$, conjointement avec l'atome de carbone voisin, représentent un noyau à 5 ou 6 chaînons, saturé ou insaturé, éventuellement mono- ou polysubstitué, qui peut contenir un ou deux hétéroatomes, en citant par exemple comme substituants: alcoyle ayant 1 à 4 atomes de carbone, halogène et hydroxy,

avec des halogénures de chloracétyle de formule:

$Hal\text{-}CO\text{-}CH_2\text{-}Cl$ (III)

dans laquelle

Hal représente du chlore ou du brome,

éventuellement en présence d'un diluant, et en ce qu'on soumet les chloracétanilides obtenues de formule:

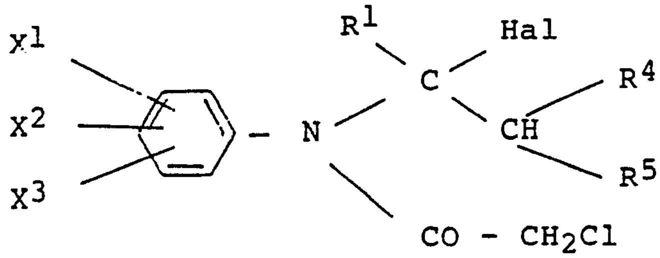
(IV)

dans laquelle
$R^1$, $R^4$, $R^5$, $X^1$, $X^2$, $X^3$ et Hal ont la signification indiquée plus haut, sans isolation préalable, le cas échéant en présence d'une base, à une éliminitation d'hydracide halogéné.

6. Agents herbicides et régulateurs de la croissance des plantes, caractérisés par une teneur en au moins une N-(1-alcényl)-chloracétanilide de formule (I).

7. Procédé pour combattre les mauvaises herbes ou pour régler la croissance des plantes, caractérisé en ce qu'on applique des N-(1-alcényl)-chloracétanilides de formule (I) sur les mauvaises herbes ou sur les plantes à régler et/ou sur leur espace vital.

8. Utilisation de N-(1-alcényl)-chloracétanilides de formule (I) pour combattre les mauvaises herbes ou pour régler la croissance des plantes.

9. Procédé de fabrication d'agents herbicides ou régulateurs de la croissance des plantes, caractérisé en ce qu'on mélange des N-(1-alcényl)-chloracétanilides de formule (I) avec des diluants et/ou des agents tensioactifs.